## Europäisches Patentamt

## European Patent Office

## Office européen des brevets

(11) Publication number: **0 352 024**
**A1**

# EUROPEAN PATENT APPLICATION

(21) Application number: **89307133.2**

(22) Date of filing: **13.07.89**

(51) Int. Cl.4: **C12P 7/02 , C12N 15/00 , C12P 1/00 , //C12P39/00, (C12P7/02,C12R1:645)**

The microorganisms have been deposited with the Culture Collection of the Commonwealth Mycologial Institute under number CMICC 326055 and 326057.

The applicant has filed a statement in accordance with Rule 28 (4) EPC (issue of a sample only to an expert). Accession number(s) of the deposit(s): CMICC 326055, 326056, 326057, 326058.

(30) Priority: **16.07.88 GB 8816996**

(43) Date of publication of application:
**24.01.90 Bulletin 90/04**

(84) Designated Contracting States:
**AT BE CH DE ES FR GB IT LI NL SE**

(71) Applicant: **The British Petroleum Company p.l.c.**
**Britannic House Moor Lane**
**London EC2Y 9BU(GB)**

(72) Inventor: **Ainsworth, Antony Martyn**
**12 Seymour Road**
**Bath BA1 6DY(GB)**
Inventor: **Rayner, Alan David Maclaren**
**81 Dovers Park**
**Bath BA1 7UD(GB)**

(74) Representative: **Ryan, Edward Terrence et al**
**BP INTERNATIONAL LIMITED Patents & Agreements Division Chertsey Road Sunbury-on-Thames Middlesex, TW16 7LN(GB)**

(54) **Production of chemical compounds.**

(57) Free chemical compounds, e.g. (+)-torreyol, are produced by culturing together on a culture medium selected unmated strains of Basidiomycete originating in different parts of the world.

EP 0 352 024 A1

## PRODUCTION OF CHEMICAL COMPOUNDS

The present invention relates to the production of chemical compounds, for example sesquiterpene compounds. In particular it relates to the production of (+)-torreyol.

The production of (+)-torreyol in cultures of the Basidiomycete Xylobolus frustulatus is disclosed by G. van Eijk et al Experimental Mycology, 8, 273-275 (1984). The (+)-torreyol was obtained by the extraction of the culture with chloroform and recrystallisation from light petroleum.

It is disclosed by M.S. Nair and M. Anchel, Lloydia, 36, 106 that the Basidiomycete Clitocybe illudens can produce crystals of (+)-torreyol directly.

Sesquiterpene alcohols such as (+)-torreyol are potentially useful chemical intermediates.

It would be desirable to find a method of producing increased quantities of chemical compounds such as (+)-torreyol.

According to the present invention the process for producing free chemical compounds comprises culturing on a culture medium extensive mycelia of two unmated strains of either a single Basidiomycete species or two closely related Basidiomycete species, said two unmated strains being cultured in contact with one another under conditions of culture medium composition, temperature and pH such as to promote growth of both mycelia and the immigration of non-self nuclei from one mycelia to the other to produce a degenerative response throughout a major part of at least one of the strains or species wherein the strains or species originate in separate regions of the world such that they do not come into contact with one another in nature, and are further selected so as to produce said degenerative interaction.

The process provides a method for providing free chemical compounds, i.e. chemical compounds which are exuded from the hyphae forming the mycelia as opposed to being retained within the hyphae.

The invention is particularly applicable to the production of (+)-torreyol.

The present invention requires the culturing in contact of extensive homokaryotic mycelial mats. Such a procedure is not natural as in nature mating occurs before extensive adjacent homokaryotic mycelial mats are formed.

The strains or species must be unmated, i.e. homokaryotic, as after mating degenerative responses are not displayed and the mated mycelia resist immigration of non-self nuclei.

Where different species are used they must be closely related i.e. they must be difficult to distinguish on taxonomic grounds. Persons skilled in culturing Basidiomycete fungi will be able to determine whether species are closely related.

The process of the present invention requires the production of a "degenerative response". By "degenerative response" we mean, throughout this specification, an interaction in which the metabolism of at least one of the strains or species is diverted into the disproportionate production of a single chemical. The normal non-degenerative response when different strains or species interact is either mating or mutual rejection along a narrow interaction interface.

The conditions under which the mycelial mats are cultured (e.g. culture medium, pH, temperature) are such as to promote a degenerative response. Thus the conditions are selected so as to produce good growth of both mats. This can be achieved using well-known conditions for culturing Basidiomycete fungi. A suitable culture medium is 2% malt extract agar. It may be necessary to culture the strains at a temperature above a threshold temperature to produce a degenerative response. Once the existence of a degenerative response has been taught by this specification the existence of a threshold temperature and its value can be determined by simple tests at various temperatures. The maximum temperature used will depend on the temperature sensitivity of the basidiomycete fungi used. Temperatures which may be used for culturing basidiomycete fungi are well known to skilled mycologists.

In the production of (+)-torreyol the temperature at which the extensive mycelia are cultured together is important. Low temperatures appear to suppress the formation of (+)-torreyol. It is preferred to use temperatures in the range 20° to 30°C e.g. 22° to 27°C.

It may be desirable to carry out the culturing step in the absence of light.

The process is carried out by culturing the mycelia on a culture medium. The medium may be in the form of a solid such as an agar gel. Alternatively the mycelia may be cultured on a solid membrane overlying a liquid medium, e.g. on a cellulose film lying on a liquid culture medium.

The culture conditions are selected such that the mycelia are actively extending when they come into contact. In particular if the mycelia are cultured on a growing medium which has a boundary, such as agar in a Petri dish, mycelia should come into contact before they come into contact with the boundary. In a standard Petri dish the mycelia may be grown from inocula spaced about 0.5 cm apart on the dish to ensure that the two mycelia are actively extending when they come into contact. On a larger dish the spacing between the inocula could be larger.

One method of culture which may be used is to culture the mycelia from inocula placed adjacent to one another on culture medium providing an elongated growth path. The elongated path may be produced using walls to partition a culture medium on a circular dish. The front between one mycelium and the other advances along the elongated path giving continuous production of the free chemical torreyl from the moving front.

Basidiomycete fungi have hyphae forming dense mats (mycelia) in which at least one nucleus (karyon) and sometimes many nuclei, may be present within a portion of the hyphae bounded by septa. Connections may be established between the hyphae forming part of unmated mycelia of two different origins leading to a transfer of karyons. This may occur between different strains of the same species. The results may be that a new stable form is established containing karyons from both strains. The usual result (mating) is that a vigorous new stable form is established containing karyons from both strains. However when strains from different parts of the world (being strains that will never normally come into contact with one another) are brought into contact, a different reaction may be observed. There may be transfer of karyons take place but there is no production of a new stable form. Instead a variable form results associated with the production of free (+)-torreyol crystals.

The production of free crystals of chemicals e.g. (+)-torreyol is readily observed so that it is easy to determine if the selected strains brought into contact meet the requirements of the invention.

We prefer to use one strain derived from Australian fungi and one strain derived from European fungi. When using Australian and European fungi we find that production of free crystals takes place on the Australian fungus. We prefer to inoculate a culture medium so that the strain producing the free crystals has a greater area available for growth than the other strain. Thus when culturing Australian and European fungi on a culture medium in a circular Petri dish we prefer to inoculate the culture excentrically so that the points of inoculation are not symmetrically disposed about the centre of the dish but are arranged so that the free growth distance of the fungus which produces crystals is greater than the free growth distance for the other fungus. The free growth distance is the distance from the point of inocculation to the boundary of the culture medium in the direction away from the point of inocculation of the other fungus.

When using a culture medium providing an elonged growth path as mentioned above it is preferred to inoculate the fungus on which crystals are produced ahead of the other fungus near one end of the path, i.e. so the point of inoculation is nearer to the other end of the elongated growth path.

By using excentric inoculation on standard Petri dishes (9 cm) we have obtained yields of up to 2.5 mg of crystal per dish.

The invention is particularly applicable to Stereum hirsutum. Particularly good results are obtained by culturing together S. hirsutum (identified as strain AF1.6 in the culture collection of the School of Biological Sciences, University of Bath) and a strain of S. hirsutum of Australian origin (identified as MP16.15 in the culture collection of the School of Biological Science of the University of Bath).

The methods of culturing mycelia of basidiomycete species with similar ecological and taxonomic characteristics to S. hirsutum are well known.

The free chemical compound, e.g. (+) torreyol, may be recovered from the culture by any convenient method. Thus torreyol may be extracted with ethanol. Water may then be added to precipitate and the torreyol which may be further purified, e.g. by sublimation, if required.

The invention will now be described with reference to the following experiments.

In these experiments the production of crystals was generally observed after incubation for 10-12 days. The maximum incubation period was four weeks.

Example 1

A homokaryotic (unmated) strain of S. hirsutum was obtained from a single basidiospore of Australian origin isolated onto an agar culture plate. The culture plate was based on 2% (w/v) malt extract agar (20g Munton and Fison spray malt A, 20g Lab M. Agar No 2 per litre distilled water). The mixture also contained 100 micrograms of novobiocin per millilitre. The strain used is identified as strain MP16.15 in the culture collection of the School of Biological Science, University of Bath.

Another mycelial culture of S. Hirsutum was prepared in the same way from a basidiospore of British origin. This was identified as strain AF1.6 in the University of Bath culture collection of the School of Biological Science of the University of Bath.

Pairings between the two strains were made by placing equally sized mycelial disc inocula (not greater

3

than 6 mm in diameter) cut from the margin of actively growing colonies. The inocula were placed approximately 1 cm apart in the centre of a 9 cm or 14 cm diameter plastic non-vented Petri dish containing 15 ml or 50 ml respectively of the malt agar. The plates were incubated in darkness at 25°C for some days. Crystals of (+)-torreyol were produced.

Example 2

The same isolation and pairing techniques were used as in Example 1, were applied to S. hirsutum strain AF1.6 (as used in Example 1) and an S. hirsutum strain of Australian origin (identified as MP16.1 in the culture collection of the School of Biological Science of the University of Bath). (+)-torreyol crystals were produced.

Example 3

Example 2 was repeated with strain AF1.6 and a strain S. hirsutum of Australian origin (identified as MP18.6 in the culture collection of the School of Biological Science of the University of Bath).
Crystals of (+)-torreyol were produced.

Comparative Examples

Numerous combinations of S. hirsutum strains from the British Isles were made following the procedure of Example 1. In no case were (+)-torreyol crystals produced.
Strains have been deposited under the Budapest Treaty at CAB International Mycological Institute (Commonwealth Mycological Institute), Kew as follows:

| Strain | Accession No |
| --- | --- |
| MP 16.15 | CMICC 326055 |
| MP 16.1 | CMICC 326056 |
| MP 18.6 | CMICC 326058 |
| AF 1.6 | CMICC 326057 |

## Claims

1. The process for producing free chemical compounds comprises culturing on a culture medium extensive mycelia of two unmated strains of either a single Basidiomycete species or two closely related Basidiomycete species, said two unmated strains being cultured in contact with one another under conditions of culture medium composition, temperature and pH such as to promote growth of both mycelia and the immigration of non-self nuclei from one mycelia to the other to produce a degenerative response throughout a major part of at least one of the strains or species wherein the strains or species originate in separate regions of the world such that they do not come into contact with one another in nature, and are further selected so as to produce said degenerative interaction.

2. The process according to claim 1 wherein free (+)-torreyol is produced.

3. The process according to either of claims 1 or 2 wherein the two strains are strains of Stereum hirsutum.

4. The process according to any of the preceding claims wherein one strain is derived from an Australian fungus and the other strain is derived from a European fungus.

5. The process according to any one of the preceding claims wherein the strains are strains of Stereum hirsutum CMICC326057 and Stereum hirsutum CMICC326055.

6. The process according to any one of claims 1 to 4 wherein the strains are Stereum hirsutum CMICC326057 and CMICC326058.

7. The process according to any one of the preceding claims wherein the culture medium is agar jelly.

8. The process according to any one of the preceding claims wherein the culture medium is a cellulose membrane above a liquid culture medium.

9. The process according to any one of the preceding claims wherein the strains are cultured at a temperature of 20 to 30° C.

10. The process according to any one of the preceding claims wherein the culture medium is inoculated with Australian S. hirsutum and with European S. hirsutum and at positions such that the Australian strain has the longest free growth path.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | JOURNAL OF GENERAL MICROBIOLOGY, vol. 135, 1989, pages 1643-1659, SGM; A.M. AINSWORTH et al.: "Hyphal and mycelial responses associated with genetic exchange within and between species of the basidiomycete genus stereum" * Page 1651, paragrapph 4 * | 1-10 | C 12 P 7/02 C 12 N 15/00 C 12 P 1/00 // C 12 P 39/00 (C 12 P 7/02 C 12 R 1:645) |
| A | TRANS. BR. MYCOL. SOC., vol. 84, no. 2, March 1985, pages 191-205; D. COATES et al.: "Genetic control and variation in expression of the 'Bow-Tie' reaction between homokaryons of stereum hirsutum" | | |
| A | TRANS. BR. MYCOL. SOC., vol. 76, no. 1, 1981, pages 41-51, The British Mycological Society, GB; D. COATES et al.: "Mating behaviour, mycelial antagonism and the establishment of individuals in stereum hirsutum" | | |
| D,A | CHEMICAL ABSTRACTS, vol. 102, no. 3, 21st January 1985, page 395, abstract no. 21182a, Columbus, Ohio, US; G.W. VAN EIJK et al.: "Isolation and identification of the sesquiterpenoid (+)-torreyol from Xylobolus frustulatus", & EXP. MYCOL. 1984, 8(3), 273-5 | | TECHNICAL FIELDS SEARCHED (Int. Cl.4) C 12 P C 12 R C 12 N |

The present search report has been drawn up for all claims

| Place of search | Date of completion of the search | Examiner |
|---|---|---|
| THE HAGUE | 03-10-1989 | DESCAMPS J.A. |